# EUROPEAN PATENT APPLICATION

(11) **EP 2 805 717 A1**
(43) Date of publication of application: **26.11.2014**
(21) Application number: 12865563.6
(22) Date of filing: 17.07.2012
(51) Int. Cl.: A61K 31/201, A23L 1/29, A61K 31/231, A61P 3/04

(54) **APPETITE SUPPRESSANT**

(30) Priority: 19.01.2012 JP 2012009387
(71) Applicant: NIPPON SUISAN KAISHA, LTD., Tokyo 100-8686 (JP)
(72) Inventor: YANG, Zhi-hong, Hachioji-shi Tokyo 192-0991 (JP); TAKEO, Jiro, Hachioji-shi Tokyo 192-0991 (JP); KATAYAMA, Masashi, Hachioji-shi Tokyo 192-0991 (JP)
(74) Representative: Vossius & Partner
(86) International application number: PCT/JP2012/068103
(87) International publication number: WO 2013/108428

(57) **Abstract**

The present invention provides an anorectic agent comprising palmitoleic acid as an active ingredient.

## Description

### TECHINICAL FIELD

The present invention relates to an anorectic agent and an intake suppressant for reducing the intake of meals, as well as a method of treating or preventing an eating disorder, in particular, bulimia. The present invention also relates to a method of controlling intake regulating hormones for inducing a sense of fullness, and a method of suppressing the intake of meals.

### BACKGROUND ART

Obesity is a risk factor for a lot of diseases including lifestyle-related diseases such as hypertension, diabetes mellitus, hyperlipidemia, and arterial sclerosis. As a way to prevent or cope with obesity, meal management for regulating food intake to the right level is currently practiced extensively.

Appetite is known to be controlled by neurotransmitters at the hypothalamus and various eating-related hormones. Known eating-related hormones include eating promoting substances such as melanin-concentrating hormone (MCH), neuropeptide Y, peptide YY, AgRP and ghrelin, and eating suppressing substances such as α-melanocyte stimulating hormone, serotonin, cholecystokinin (CCK), and glucagon-like peptide-1 (GLP-1). Cholecystokinin is a peptide hormone composed of 33 amino acids which is secreted typically from the duodenum. Cholecystokinin promotes the contraction of the gall bladder and the secretion of digestive enzymes while at the same time it is involved in the transmission of satiety signals via the central and peripheral nerve systems. An increase in blood levels of cholecystokinin induces a sense of fullness which in turn suppresses the eating activity (Non-Patent Documents 1-4).

When patients on obesity treatment restrict food intake, they will inevitably feel a strong appetite due to secretion of easting-related hormones and this has been a big problem with the management of meal size. Even if the desired weight loss is achieved, the patient will sense a strong appetite if hormones that promote eating are secreted excessively and this has not only resulted in weight rebound but has also created a strong discomfort and stress, eventually causing disorders in daily life.

Known as appetite reducers are drugs that produce a sense of increased alertness and exert a sympathetic action, as exemplified by amphetamine-type drugs, fenfluramine, mazindol, and fentermine. Of these, mazindol is the only drug that is approved for manufacture in Japan but it is known to have side actions such as pulmonary hypertension and various psychoneurotic symptoms. Reports about other anorectic agents are also known (Patent Documents 1-7).

Fatty acids are constituent elements of fat as an essential nutrient and various fatty acids are known to occur naturally. Certain fatty acids have been reported to have physiological activity. Concerning effects on the digestive tract, it has been reported that upon oral administration of certain fatty acids, the residence time of nutrients in the small intestine is extended to enhance their digestion and absorption (Patent Document 8).

It has also been reported that ingestion of short-chained monounsaturated fatty acids improves lipid metabolism to reduce fat deposition in the liver (Patent Document 9). Among the monounsaturated fatty acids, palmitoleic acid (C16:1, n-7) has been reported to be effective in enhancing the action of insulin in the skeletal muscle to suppress the occurrence of fat liver (Non-Patent Documents 5-7).

### CITATION LIST

### PATENT LITERATURE

Patent Document 1: JP Hei 7-145054A
Patent Document 2: JP Hei 9-20675A
Patent Document 3: JP2007-519605A
Patent Document 4: JP2008-201683A
Patent Document 5: JP2009-51770A
Patent Document 6: JP2009-209096A
Patent Document 7: JP2011-239774A
Patent Document 8: JP Hei 11-505258A
Patent Document 9: JP Hei 5-508854A

### NON-PATENT LITERATURE

Non-Patent Document 1: J. Clin. Invest., 1986, 77, 992-996;
Non-Patent Document 2: Am. J. Physiol. Regul. Integr. Comp. Physiol., 2000, 279, 189-195;
Non-Patent Document 3: Diabetes Care, 2003, 26, 2929-2940;
Non-Patent Document 4: J. Clin. Endocrinol. Metab., 1990, 70, 1312-1318;
Non-Patent Document 5: Biochem. J., 2006, 399, 473-481;
Non-Patent Document 6: Cell, 2008, 134, 933-944;
Non-Patent Document 7: Lipids in Health and Disease, 2011, 10, 120

### SUMMARY OF INVENTION

### TECHNICAL PROBLEM

People who are under meal intake control for prevention or treatment of obesity will inevitably have a strong sense of appetite from the effects of eating-related hormones. Such enhanced appetite is not only a substantial bar to meal management but it also presents a great stress to those under the meal management, causing deterioration in the quality of their daily life. Drugs such as mazindol are known as means for reducing appetite, but given reports on side actions and other difficulties, problems with the safety of their use have been pointed out. It is therefore desired to develop anorectic agents that are safe to use.

An object of the present invention is to provide an anorectic agent that is safe to use.

### SOLUTION TO PROBLEM

In order to attain this object, the present inventors made intensive studies and found that palmitoleic acid or esters thereof have a satisfactory appetite suppressing effect. The present invention has been completed on the basis of this finding.

According to one aspect of the present invention, there are provided anorectic agents as set forth below under (1) to (5).
(1) An anorectic agent comprising a component selected from among palmitoleic acid, salts thereof, and esters thereof as an active ingredient.
(2) The anorectic agent as recited in (1) above, which comprises as the active ingredient a palmitoleic acid ester selected from C₁₋₆ alkyl esters of palmitoleic acid and glycerides comprising palmitoleic acid as a constituent fatty acid.
(3) The anorectic agent as recited in (1) above, which comprises an ethyl ester of palmitoleic acid as the active ingredient.
(4) The anorectic agent as recited in (1) above, which comprises as the active ingredient a triglyceride comprising palmitoleic acid as a constituent fatty acid.
(5) The anorectic agent as recited in (4) above, wherein palmitoleic acid accounts for 30% or more of the fatty acid composition of the triglyceride.
   According to another aspect of the present invention, there are provided pharmaceutical compositions as set forth below under (6) to (10).
(6) A pharmaceutical composition for use in treatment or prevention of eating disorder or obesity, which comprises a component selected from among palmitoleic acid, salts thereof, and esters thereof as an active ingredient.
(7) The pharmaceutical composition as recited in (6) above, which comprises as the active ingredient a palmitoleic acid ester selected from C₁₋₆ alkyl esters of palmitoleic acid and glycerides comprising palmitoleic acid as a constituent fatty acid.
(8) The pharmaceutical composition as recited in (6) above, which comprises an ethyl ester of palmitoleic acid as the active ingredient.
(9) The pharmaceutical composition as recited in (6) above, which comprises as the active ingredient a triglyceride comprising palmitoleic acid as a constituent fatty acid.
(10) The pharmaceutical composition as recited in (9) above, wherein palmitoleic acid accounts for 30% or more of the fatty acid composition of the triglyceride.
   According to another aspect of the present invention, there are provided therapeutic methods as recited below under (11) to (15).
(11) A method for treating eating disorder or obesity, which comprises administering a therapeutically effective amount of palmitoleic acid, a salt thereof or an ester thereof to a subject.
(12) The method as recited in (11) above, which comprises administering a palmitoleic acid ester selected from C₁₋₆ alkyl esters of palmitoleic acid and glycerides comprising palmitoleic acid as a constituent fatty acid.
(13) The method as recited in (11) above, which comprises administering an ethyl ester of palmitoleic acid.
(14) The method as recited in (11) above, which comprises administering a triglyceride comprising palmitoleic acid as a constituent fatty acid.
(15) The method as recited in (14) above, wherein palmitoleic acid accounts for 30% or more of the fatty acid composition of the triglyceride.
   According to another aspect of the present invention, there are provided methods for suppressing an amount of ingestion as recited below under (16) to (21).
(16) A method for suppressing an amount of ingestion by a subject, which comprises administering a therapeutically effective amount of palmitoleic acid, a salt thereof or an ester thereof to the subject.
(17) The method as recited in (16) above, which comprises administering a palmitoleic acid ester selected from C₁₋₆ alkyl esters of palmitoleic acid and glycerides comprising palmitoleic acid as a constituent fatty acid.
(18) The method as recited in (16) above, which comprises administering an ethyl ester of palmitoleic acid.
(19) The method as recited in (16) above, which comprises administering a triglyceride comprising palmitoleic acid as a constituent fatty acid.
(20) The method as recited in (19) above, wherein palmitoleic acid accounts for 30% or more of the fatty acid composition of the triglyceride.
(21) The method as recited in any one of (16) to (19) above, which comprises administering a therapeutically effective amount of palmitoleic acid, a salt thereof or an ester thereof to the subject before meals.
   According to another aspect of the present invention, there are provided foods and beverages as recited below under (22) to (29).
(22) A food or beverage comprising a component selected from among palmitoleic acid, salts thereof, and esters thereof.
(23) The food or beverage as recited in (22) above, which comprises as an active ingredient a palmitoleic acid ester selected from C₁₋₆ alkyl esters of palmitoleic acid and glycerides comprising palmitoleic acid as a constituent fatty acid.
(24) The food or beverage as recited in (22) above, which comprises an ethyl ester of palmitoleic acid as an active ingredient.
(25) The food or beverage as recited in (22) above, which comprises as an active ingredient a triglyceride comprising palmitoleic acid as a constituent fatty acid.
(26) The food or beverage as recited in (25) above, wherein palmitoleic acid accounts for 30% or more of the fatty acid composition of the triglyceride.
(27) The food or beverage as recited in any one of (22) to (26) above, which is to be used as a food or beverage for patients suffering from obesity or eating disorder.
(28) The food or beverage as recited in any one of (22) to (26) above, which is to be used as a food or beverage for preventing obesity or eating disorder.
(29) The food or beverage as recited in any one of (22) to (28) above, which contains 0.01 to 99 wt% of the component selected from among Palmitoleic acid, salts thereof, and esters thereof.

### ADVANTAGEOUS EFFECTS OF INVENTION

According to the present invention, there are provided methods for coping with eating disorders (bulimia, in particular) or disorders attributable to obesity. The present invention particularly provides therapeutics or prophylactics for eating disorders (bulimia, in particular) or obesity that are highly safe and which are suitable for continued ingestion as foods, beverages, dietary supplements and the like.

### BRIEF DESCRIPTION OF DRAWINGS

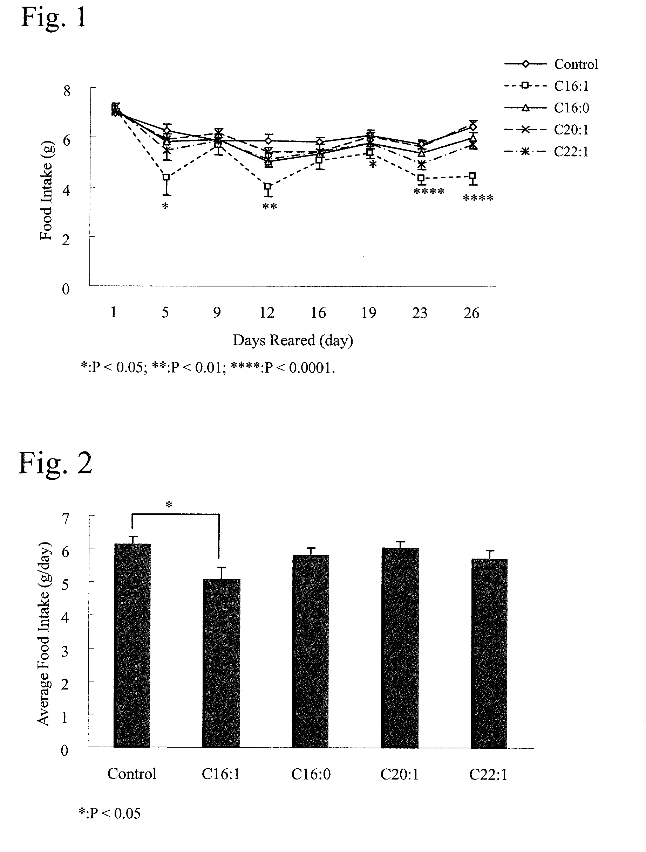

Fig. 1 is a graph showing changes in the amount of food intake by KKAy mice after the administration of palmitoleic acid and other fatty acids; "Control" in the graph represents the profile of the amount of food intake by a control group; "C 16:1" refers to a palmitoleic acid (C16:1) administered group; "C16:0" refers to a palmitic acid (C16:0) administered group; "C20:1" refers to a gadoleic acid (C20:1) administered group; "C22:1" refers to an erucic acid (C22:1) administered group (the same definitions apply in Figs. 2 to 4); as for statistical significance over the control group, *, ** and **** mean P < 0.05, P < 0.01, and P < 0.0001, respectively.
Fig. 2 is a graph showing daily averages for the amount of food intake by KKAy mice after the administration of palmitoleic acid and other fatty acids; a significant drop in the amount of food intake was observed only in the palmitoleic acid (C16:1) administered group (*: P < 0.05).

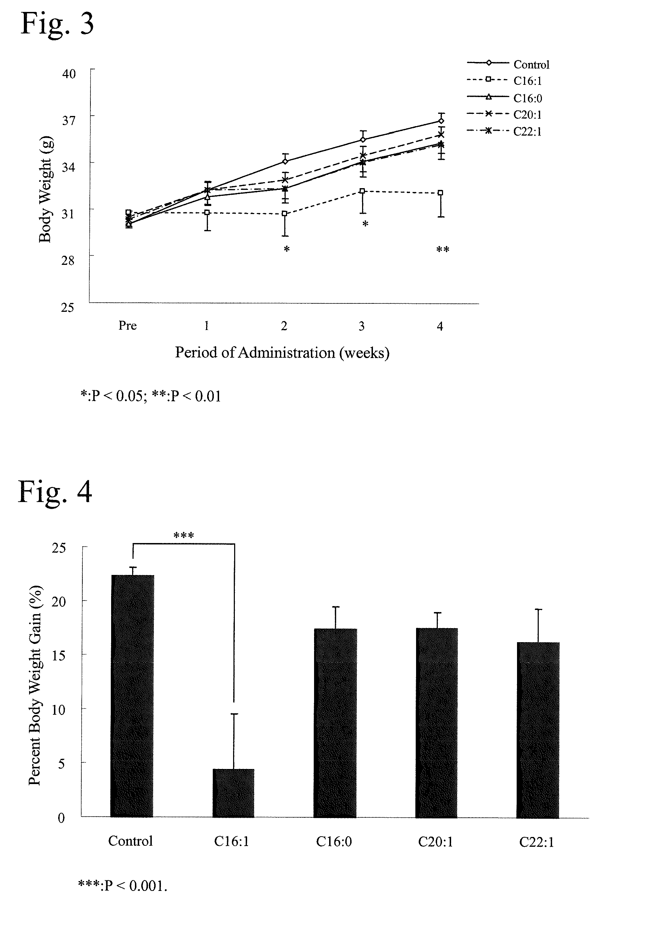

Fig. 3 is a graph showing changes in the body weight of KKAy mice after the administration of palmitoleic acid and other fatty acids; as for statistical significance over the control group, * and ** mean P < 0.05 and P < 0.01, respectively.
Fig. 4 is a graph showing percent body weight gains in KKAy mice 4 weeks after the administration of palmitoleic acid and other fatty acids; a significant drop in the percent body weight gain was observed only in the palmitoleic acid (C16:1) administered group (***: P < 0.001).

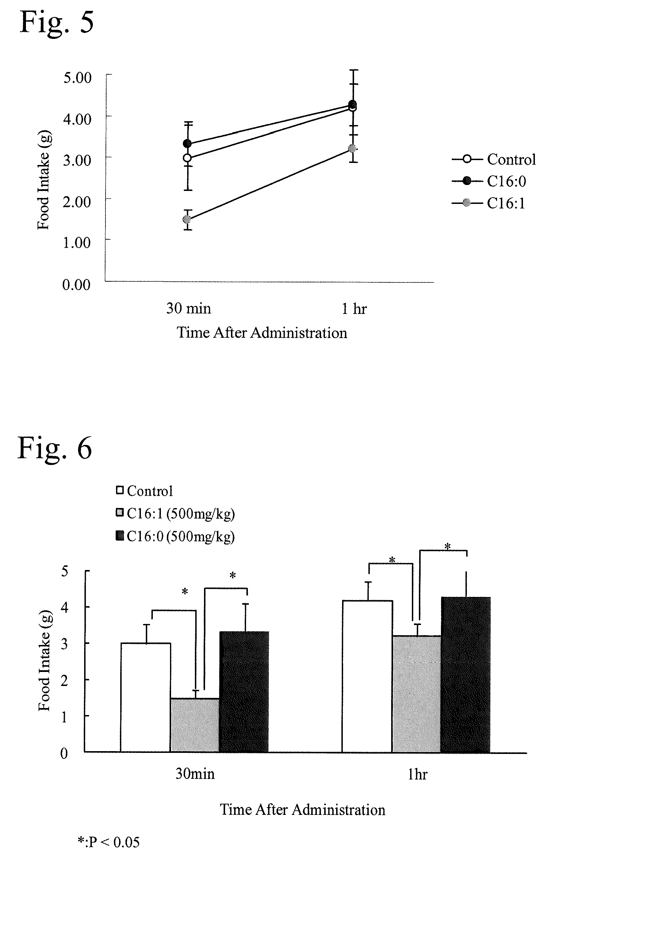

Fig. 5 is a graph showing the amount of food intake by SD rats both 30 minutes and an hour after the administration of palmitoleic acid and palmitic acid; "Control" in the graph refers to a control group; "C16:1" refers to a palmitoleic acid (C16:1) administered group (500 mg/kg); and "C16:0" refers to a palmitic acid (C16:0) administered group (500 mg/kg); the same definitions apply in Fig. 6.
Fig. 6 is a graph showing the amount of food intake by SD rats both 30 minutes and an hour after the administration of palmitoleic acid and palmitic acid; as for statistical significance, * means P < 0.05.

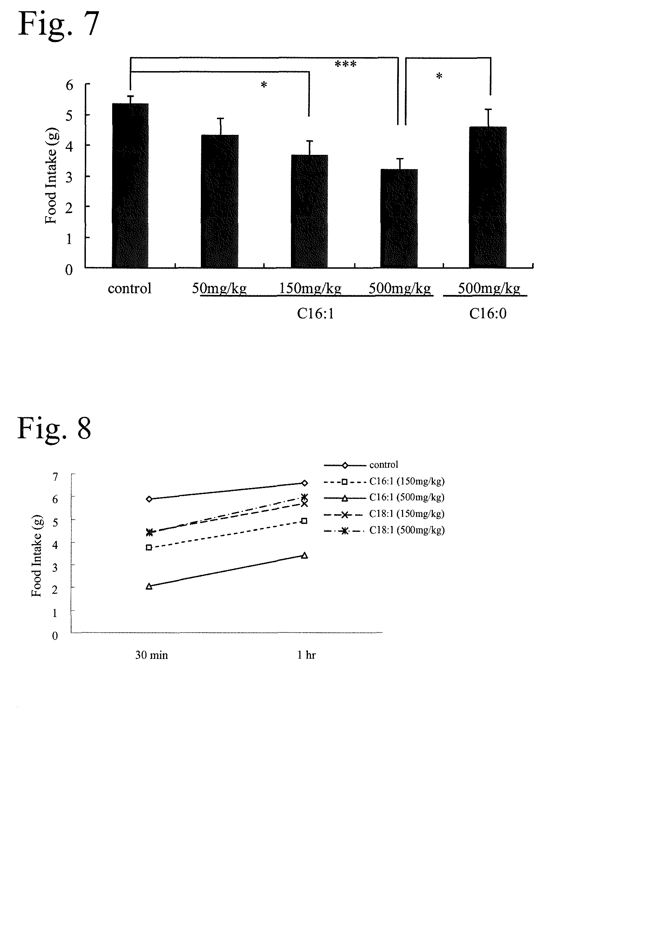

Fig. 7 is a graph showing the amount of food intake by SD rats 30 minutes after the administration of palmitoleic acid and palmitic acid; "Control" in the graph refers to a control group; "C16:1" refers to a palmitoleic acid (C16:1) administered group (50 mg/kg, 150 mg/kg, or 500 mg/kg); and "C16:0" refers to a palmitic acid (C16:0) administered group (500 mg/kg); as for statistical significance, * and *** mean P < 0.05 and P < 0.001, respectively.

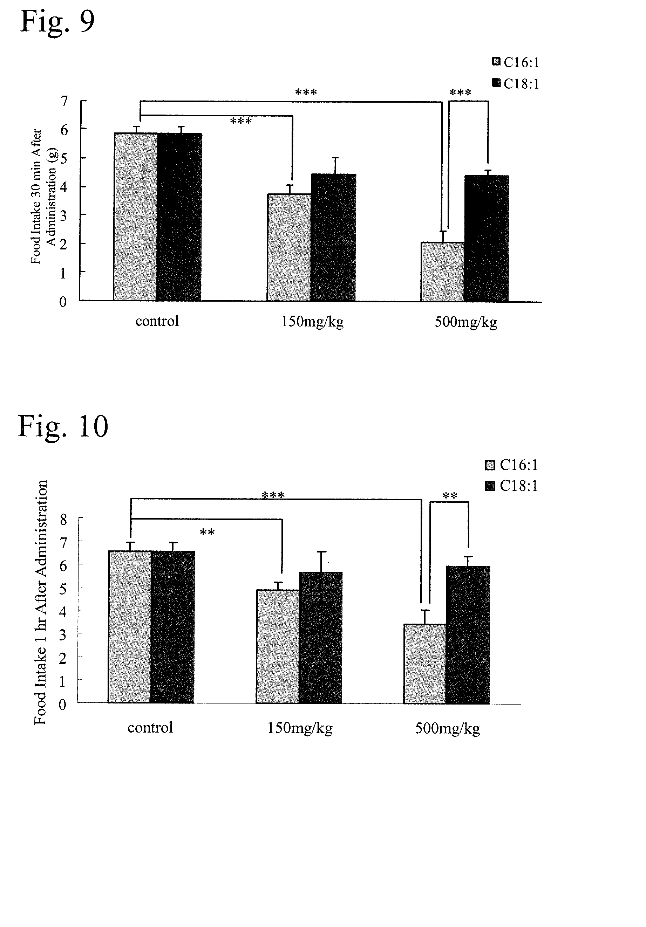

Fig. 8 is a graph showing the amount of food intake by SD rats both 30 minutes and an hour after the administration of palmitoleic acid and oleic acid; "Control" in the graph refers to a control group; "C16:1" refers to a palmitoleic acid (C16:1) administered group (150 mg/kg or 500 mg/kg); and "C18:1" refers to an oleic acid (C18:1) administered group (150 mg/kg or 500 mg/kg); the same definitions apply in Figs. 9 and 10.
Fig. 9 is a graph showing the amount of food intake by SD rats 30 minutes after the administration of palmitoleic acid and oleic acid; as for statistical significance, *** means P < 0.001.
Fig. 10 is a graph showing the amount of food intake by SD rats an hour after the administration of palmitoleic acid and oleic acid; as for statistical significance, ** and *** mean P < 0.01 and P < 0.001, respectively.

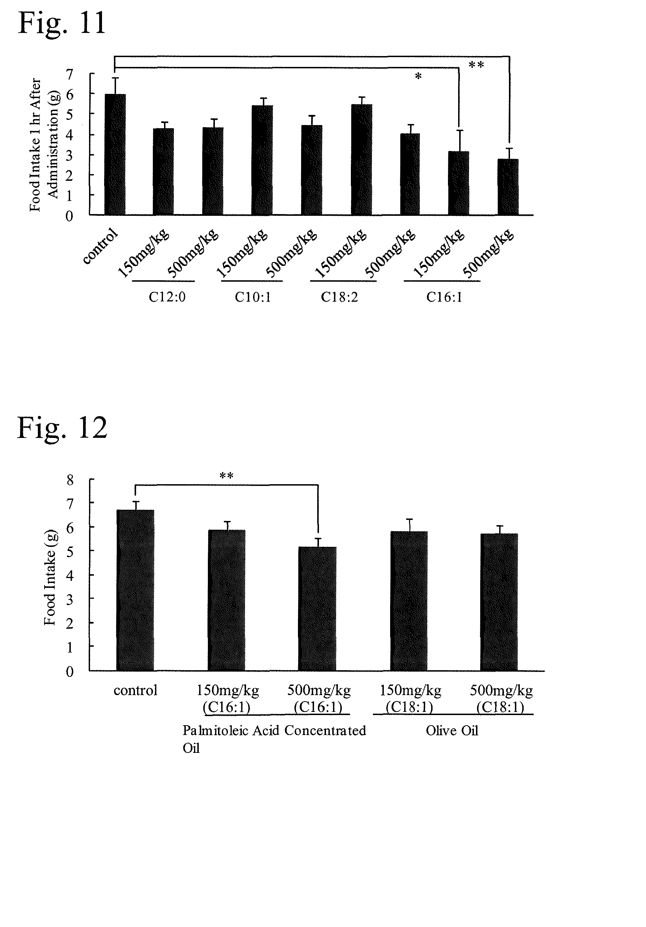

Fig. 11 is a graph showing the amount of food intake by SD rats an hour after the administration of palmitoleic acid and other fatty acids; "Control" in the graph refers to a control group; "C12:0" refers to a lauric acid (C12:0) administered group (150 mg/kg or 500 mg/kg); "C10:1" refers to a decenoic acid (C10:1) administered group (150 mg/kg or 500 mg/kg); "C18:2" refers to a linoleic acid (C18:2) administered group (150 mg/kg or 500 mg/kg); and "C16:1" refers to a palmitoleic acid (C16:1) administered group (150 mg/kg or 500 mg/kg); as for statistical significance, * and ** mean P < 0.05 and P < 0.01, respectively.
Fig. 12 is a graph showing the amount of food intake by SD rats an hour after the administration of a palmitoleic acid concentrated oil and olive oil; the graph shows the results with a control group (Control), a palmitoleic acid concentrated oil administered group (150 mg/kg or 150 mg/kg as calculated for the palmitoleic acid (C16:1) in the oil), and an olive oil administered group (150 mg/kg or 150 mg/kg as calculated for the oleic acid in the oil); as for statistical significance, ** means P < 0.01.

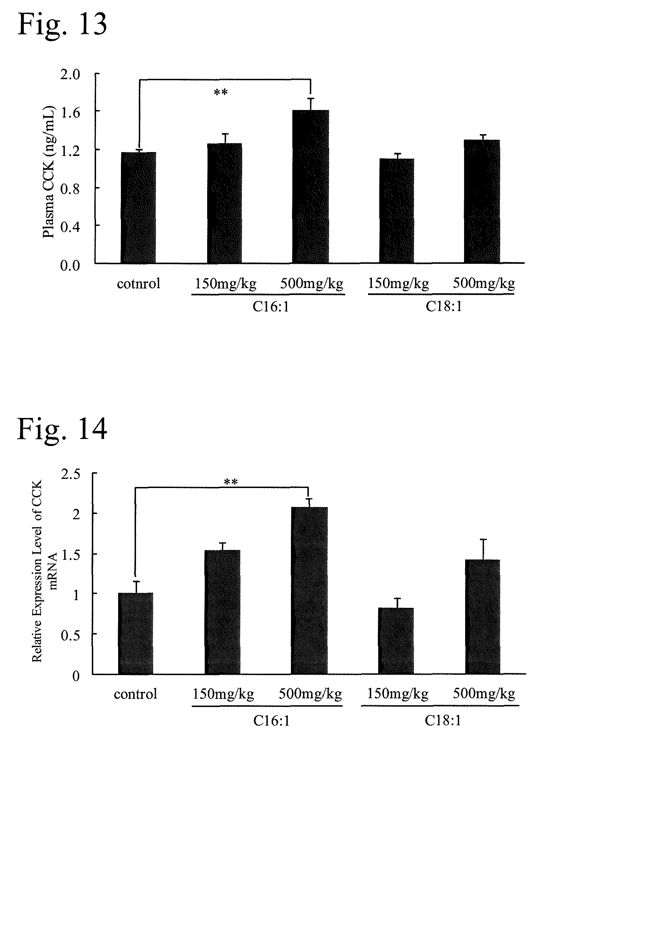

Fig. 13 is a graph showing the blood CCK level in SD rats an hour after the administration of palmitoleic acid and oleic acid; "Control" in the graph refers to a control group; "C16:1" refers to a palmitoleic acid (C16:1) administered group (150 mg/kg or 500 mg/kg); and "C18:1" refers to an oleic acid (C18:1) administered group (150 mg/kg or 500 mg/kg); the same definitions apply in Fig. 14; as for statistical significance, ** means P < 0.01.
Fig. 14 is a graph showing the small intestine CCK mRNA expression level in SD rats an hour after the administration of palmitoleic acid and oleic acid; as for statistical significance, ** means P < 0.01.

### DESCRIPTION OF EMBODIMENTS

On the following pages, the present invention will be described more specifically.

The palmitoleic acid, salts thereof and esters thereof that are to be used in the present invention are not particularly limited and they may be of any types that can be used in pharmaceuticals or foods. The glycerides containing palmitoleic acid as a constituent fatty acid can be produced by known production methods, such as the one described in JP 2007-70486A, using natural oils and fats (say, seal oil and macadamia nut oil) as the starting material. Free palmitoleic acid, salts thereof, and esters other than glycerides thereof can be prepared by known methods, starting from such glycerides, for example. Palmitoleic acid, salts thereof and esters thereof that have been prepared by other methods such as culture of microorganisms can also be used.

Exemplary palmitoleic acid esters that may be used in the present invention include C₁₋₆ alkyl esters (e.g. methyl ester, ethyl ester, n-propyl ester, i-propyl ester, n-butyl ester, s-butyl ester, t-butyl ester, n-pentyl ester, n-hexyl ester, etc.) of palmitoleic acid, glycerides containing palmitoleic acid as a constituent fatty acid, and so forth. Preferred esters include ethyl palmitoleate and glycerides containing palmitoleic acid as a constituent fatty acid.

In one mode of the present invention, oils or fats that contain palmitoleic acid esters may be used as a component. From the viewpoint of ingestion's efficiency, it is preferred that the palmitoleic acid esters to be used have higher purities. The proportion of palmitoleic acid to all fatty acids contained in oils and fats as esters or free fatty acids may be at least 10%, preferably at least 20%, and more preferably at least 99%. If ethyl palmitoleate is to be used, its purity as it is present in the oil or fat used may be at least 70 wt%, preferably at least 90 wt%, and more preferably at least 99 wt%.

The glycerides containing palmitoleic acid as a constituent fatty acid may be any one of monoglyceride, diglyceride, and triglyceride, or mixtures thereof. For example, the glycerides to be used may contain other constituent fatty acids, such as palmitic acid, stearic acid, oleic acid, linoleic acid, arachidonic acid, eicosapentaenoic acid, docosahexaenoic acid, etc. The proportion of palmitoleic acid to all fatty acids contained as constituent fatty acids in the glycerides may be at least 10%, preferably at least 20%, and more preferably at least 30%.

The amounts of the palmitoleic acid, salts thereof and esters thereof that are to be ingested by a subject in the present invention are not particularly limited and they may, for example, be ingested in amounts at least equal to the effective amount required for attaining the intended effect. The "effective amount" as mentioned above refers to the quantity required for exhibiting the appetite suppressing action. To give exemplary values, the effective amount is 10-2000 mg/kg, preferably 50-1000 mg/kg, and particularly preferably 150-500 mg/kg, daily per kg of an animal's body weight. Particularly in the case of human adults, the effective amount is 10-10000 mg/50 kg of body weight, preferably 50-5000 mg/50 kg of body weight, more preferably 100-1000 mg/50 kg of body weight, and particularly preferably 150-500 mg/50 kg of body weight, per day. In the case of human adults, greater amounts are preferably ingested in order to attain more marked effects; on the other hand, too much intake of oils and fats generally imposes greater burden on the gastrointestinal system, resulting in unfavorable conditions such as heavy stomach feeling. The amounts of ingestion listed above may be values for single intake, or for several intakes, such as two or three.

The present invention can be used as an appetite reducer for various purposes, such as suppression of food intake, alleviation of discomfort or stress due to appetite, treatment or prevention of alimentary diseases due to appetite (e.g. gastritis, gastric ulcer, duodenal ulcer, etc.), protection of alimentary organs through adjustment of digestive hormone secretions, and suppression of body weight loss or gain. For example, the appetite reducer of the present invention can be ingested on an empty stomach for various purposes, such as alleviation of stress through appetite suppression, as well as protection of alimentary organs and treatment or prevention of alimentary diseases through suppression of digestive enzyme secretions. The appetite reducer of the present invention can be ingested before, during or after meal for such purposes as suppression of intake through appetite suppression. In one aspect of the present invention, an intake suppressor is provided that comprises a component selected from among palmitoleic acid, salts thereof, and esters thereof as an active ingredient.

The present invention may also be applied to eating disorders, in particular, disorders due to food cravings and bulimia.

The therapeutic or prophylactic of the present invention may optionally contain such components as known colorants, preservatives, fragrances, flavors, coating agents, antioxidants, vitamins, amino acids, peptides, proteins, and minerals (e.g. iron, zinc, magnesium, iodine).

Examples of the antioxidants referred to hereinabove include tocopherol, dry yeasts, glutathione, lipoic acid, quercetin, catechin, coenzyme Q10, enzogenol, proanthocyanidins, anthocyanidin, anthocyanin, carotenes, lycopene, flavonoid, reseveratrol, isoflavones, zinc, melatonin, ginkgo leaf, *Alpinia speciosa,* hibiscus, vitamins of C group, and extracts thereof.

Antioxidants can also function as oxidation preventing agents for enhancing the storage stability of the palmitoyl acid, salts thereof or esters thereof, or other unsaturated fatty acids as they are present as ingredients. As a specific mode, tocopherol may be so incorporated that it accounts for 0.01-3 wt%, preferably 0.1-1.0 wt%, more preferably 0.1-0.5 wt%, as relative to the fat or oil containing unsaturated fatty acids.

Examples of vitamins include: vitamins of A group (e.g. retinal, retinol, retinoic acid, carotene, dehydroretinal, lycopene, and salts thereof); vitamins of B group (e.g. thiamine, thiamine disulfide, dicetiamine, octotiamine, cycotiamine, bisibuthiamine, bisbentiamine, prosultiamine, benfotiamine, fursultiamine, rivoflavin, flavinadenine dinucleotide, pyridoxine, pyridoxal, hydroxocobalamin, cyanocobalamin, methylcobalamin, deoxyadenocobalamin, folic acid, tetrahydrofolic acid, dihydrofolic acid, nicotinic acid, nicotinic acid amide, nicotinic alcohol, pantothenic acid, panthenol, biotin, choline, inositol, pangamic acid, and salts thereof; vitamins of C group (e.g. ascorbic acid and derivatives thereof, erythorbic acid and derivatives thereof, as well as pharmacologically acceptable salts thereof); vitamins of D group (e.g. ergocalciferol, colecarciferol, hydroxycolecarciferol, dihydroxycolecarciferol, dihydrotachysterol, and pharmacologically acceptable salts thereof); vitamins of E group (e.g. tocopherol and derivatives thereof, ubiquinone derivatives, and pharmacologically acceptable salts thereof); and other vitamins (e.g. carnitine, ferulic acid, γ-oryzanol, orotic acid, rutin (vitamin P), eriocitrin, hesperidin, and pharmacologically acceptable salts thereof).

Examples of amino acids include leucine, isoleucine, valine, methionine, threonine, alanine, phenylalanine, tryptophan, lysine, glycine, asparagine, aspartic acid, serine, glutamine, glutamic acid, proline, tyrosine, cysteine, histidine, ornithine, hydroxyproline, hydroxylysine, glycylglycine, aminoethylsulfonic acid (taurine), cystine, and pharmacologically acceptable salts thereof.

The pharmaceutical composition, therapeutic or prophylactic of the present invention may be formulated in any forms suitable for pharmaceutical compositions, functional foods, health foods, beverages, dietary supplements, etc., as exemplified by various solid preparations such as granules (including dry syrups), capsules (soft capsules and hard capsules), tablets (including chewables, etc.), powders (dusts), pills, etc. and liquid preparations such as liquids for internal use (including liquids, suspensions, and syrups). The therapeutic or prophylactic of the present invention which typically takes the form of an appetite reducer may also be used on its own as a pharmaceutical composition, functional food, health food, dietary supplement, etc..

Examples of additives that may be used to formulate pharmaceutical preparations include excipients, lubricants, binders, disintegrants, fluidization agents, dispersants, wetting agents, antiseptics, viscous agents, pH modifiers, colorants, corrigents, surfactants, and solubilizing agents. When the intended form is a liquid, thickening agents such as pectin, xanthan gum and guar gum may be incorporated. Coating agents may be used to formulate coated tablets or paste of gels. Still other forms may be formulated in accordance with known methods.

Moreover, the therapeutic or prophylactic of the present invention may be used as various foods and beverages including drinks, confectioneries, bread, and soups, or as additives that are to be contained therein. The processes for producing these foods and beverages are not particularly limited unless they are deleterious to the effects of the present invention and they may be produced in accordance with any methods that are employed by skilled artisans in specific applications.

When the present invention takes the form of foods and beverages, the applicable foods and beverages are not particularly limited and may include, for example, common retort foods, frozen foods, instant foods (e.g. noodles), canned foods, and sausages, as well as cookies, biscuits, cereal bars, crackers, snacks (e.g. potato chips), pastry, cakes, pies, candies, chewing gums (including pellets and sticks), jelly, soups, ice creams, dressings, and yogurt; also included are dietary supplements in such forms as tablets, capsules and emulsions, as well as soft drinks.

In the case where the present invention takes the form of foods and beverages, the content of the ingredient selected from among palmitoleic acid, salts thereof and esters thereof may account for 0.01-99 wt%, preferably 1-50 wt%, more preferably 10-30 wt%, of the total amount of the food or beverage.

Selling the product according to the present invention, with the therapeutic or preventive effects of the present invention being claimed on its package container, the instructions that come with it, or an associated pamphlet, is within the scope of the present invention. In addition, advertising and selling the product according to the present invention, with its effects being claimed on TV, Internet websites, pamphlets, newspapers, magazines, etc., are also within the scope of the present invention.

### EXAMPLES

The present invention will now be described specifically by means of the following examples, which should in no way be taken to limit the scope of the present invention.

### [Example 1] Effects of Long-Term Oral Administration of Palmitoleic Acid (Free Fatty Acid) on the Food Intake and Body Weight of KKAy Mice (Male)

### (1) Preparing dosing samples

Using a 1.5% (by weight) aqueous solution of a fatty acid ester of glycerol (RYOTO®POLYGLYESTER; product of Mitsubishi-Kagaku Foods Corporation) as a solvent, a free fatty acid form of palmitoleic acid (C16:1), a free fatty acid form of palmitic acid (C16:0), a free fatty acid form of gadoleic acid (C20:1), and a free fatty acid form of erucic acid (C22:1), all being products of Sigma with purities of 99% and more, were added to the solvent and uniformly emulsified by sonication in an ice bath to thereby prepare dosing samples.

### (2) Diabetic model animal

Male, spontaneously diabetic model mice KKAy/Ta (hereinafter referred to as KKAy mice) were used in the test. Five-week-old KKAy mice (CLEA Japan, Inc.) were purchased and preliminarily reared for a week in individual cages. During the preliminary rearing period, the animals were allowed free access to the solid feed Labo MR Stock (Nosan Corporation) and distilled water through water bottles.

### (3) Main test with KKAy mice

After the preliminary rearing, the 6-week-old KKAy mice were divided into the following five groups (10 animals per group) considering their body weight: a control group administered with only the solvent (hereinafter referred to as "control group"); a group administered with palmitoleic acid (C16:1) (hereinafter "C16:1 administered group"); a group administered with palmitic acid (C16:0) (hereinafter "C16:0 administered group"); a group administered with gadoleic acid (C20:1) (hereinafter "C20:1 administered group"); and a group administered with erucic acid (C22:1) (hereinafter "C22:1 administered group"); these groups of mice were subjected to the main test. In the main test, the mice of each group were reared for 4 weeks under the environment of light (12 hr) and dark (12 hr) cycles as they were allowed free access to the powdered feed Labo MR Stock (Nosan Corporation) and distilled water through water bottles. In the process, the solvent or each fatty acid under test was orally administered through a gastric tube at 10 a.m. every day. To the control group of KKAy mice, the solvent was orally administered at a dose of 10 mL/kg per animal. The test subjects, C16:1, C16:0, C20:1 and C22:1, each weighing 300 mg, were added to 10 mL of the solvent, emulsified and orally administered to the KKAy mice at a dose of 10 mL/kg per animal.

### (4) Measuring the food intake and body weight of KKAy mice

During the 4-week rearing period, the amount of food supplied was measured at days 1, 5, 9, 12, 16, 19, 23 and 26 after dosing and the amount of leftover was measured on the next day, with the respective measurements being conducted for each feeder. The amount of daily food intake was calculated from the difference between the values for two consecutive measurements. The "average food intake" is the average of the food intakes as measured on the respective days of measurement. Body weight measurement was conducted on the day when dosing started (Pre) as well as 1, 2, 3 and 4 weeks after the dosing. The percent body weight gain was calculated by the formula [(final body weight - initial body weight)/initial body weight] x 100. The "final body weight" is the body weight as measured after the end of the 4-week rearing period whereas "initial body weight" is the body weight as measured at the start of the test.

### (5) Results of Changes in Food Intake and Body Weight

Compared with the control group, the C16:1 administered group experienced significant drops in the food intake (Figs. 1 and 2). Concerning body weight change, as compared with the control group, the C16:1 administered group experienced a significant drop in body weight, starting from the second to the fourth week of dosing (Fig. 3). Compared with the control group, the C16:1 administered group experienced a significant drop in the percent body weight gain (Fig. 4). In contrast, the groups administered with the other fatty acids were found to have such a tendency that both the food intake and the body weight dropped slightly, but no significant difference was observed.

### [Example 2] Study with SD Rats (Male) of the Effect on Food Intake of Short-Period Administration of Palmitoleic Acid (Free Fatty Acid)

### (1) Preparing dosing samples

Using a 1.5% (by weight) aqueous solution of a fatty acid ester of glycerol (RYOTO® POLYGLYESTER; product of Mitsubishi-Kagaku Foods Corporation) as a solvent, a free fatty acid form of palmitoleic acid (C16:1), a free fatty acid form of palmitic acid (C16:0), a free fatty acid form of oleic acid (C18:1), a free fatty acid form of lauric acid (C12:0), a free fatty acid form of decenoic acid (C10:1), and a free fatty acid form of linoleic acid (C18:2), all being products of Sigma, were added to the solvent and uniformly emulsified by sonication in an ice bath to thereby prepare dosing samples.

### (2) Experimental animal

Male Sprague-Dawley rats (hereinafter referred to as SD rats) were used in the experiment. Nine-week-old SD rats (Japan SLC, Inc.) were purchased and preliminarily reared for a week in individual cages. During the preliminary rearing period, the animals were allowed free access to the solid feed Labo MR Stock (Nosan Corporation) and distilled water through water bottles.

### (3) Main test with SD rats

After the preliminary rearing, the 10-week-old SD rats were divided into the following seven groups (10 animals per group) considering their body weight: a control group administered with only the solvent (hereinafter referred to as "control group"); a group administered with palmitoleic acid (C16:1) (hereinafter "C16:1 administered group"); a group administered with palmitic acid (C16:0) (hereinafter "C16:0 administered group") ; a group administered with oleic acid (C18:1) (hereinafter "C18:1 administered group") ; a group administered with lauric acid (C12:0) (hereinafter "C12:0 administered group"); a group administered with decenoic acid (C10:1) (hereinafter "C10:1 administered group"); and a group administered with linoleic acid (C18:2) (hereinafter "C18:2 administered groups"); these groups of rats were subjected to the main test. In the main test, the solvent or each fatty acid under test was orally administered in a single dose through a gastric tube on the very day of the experiment. To the control group of SD rats, the solvent was orally administered at a dose of 10 mL/kg per animal. The test subjects, C16:1, C16:0, C18:1, C12:0, C10:1 and C18:2, each taken in an amount of 150 mg or 500 mg, were added to 10 mL of the solvent and emulsified; the emulsion was orally administered to the SD rats in a dose of 10 mL/kg per animal and a measurement was conducted both 30 minutes and an hour later. The amount of food intake (the amount of food supplied immediately after the dosing of sample minus the amount of food at a specified time after the dosing of sample) was measured both 30 minutes and an hour after oral administration of the test sample.

### (4) The effect on food intake of single-dose oral administration of palmitoleic acid (free fatty acid)

Both thirty minutes and an hour after administration of the test substance, the C16:1 administered group experienced a significant drop in food intake compared with the control group and the C16:0 administered group (Figs. 5 and 6). In the test for dose-dependency of C16:1, a measurement conducted 30 minutes after administration of the test substance showed that the food intake by the C16:0 administered group decreased significantly in a dose-dependent manner as compared with the control group and the C16:0 administered group (Fig. 7). In the test of comparison with C18:1 which is also a monounsaturated fatty acid, the C16:1 administered group experienced significant drops in food intake compared with the control group and the C18:1 administered group, as measured both 30 minutes and an hour after administration of the test substance (Figs. 8-10). Moreover, in the tests of comparison with the other fatty acids, i.e., a short-chain saturated fatty acid (C12:0), a short-chain monounsaturated fatty acid (C10:1) and a long-chain polyunsaturated fatty acid (C18:2), the C16:1 administered group experienced significant drops in food intake compared with the control group when measured an hour after administration of the test substance but there were no significant drops in the food intake by the C12:0 administered group, the C10:1 administered group, and the C18:2 administered group (Fig. 11).

### [Example 3] The Effect on Food Intake by SD Rats (Male) of Administering Triglyceride Palmitoleate Concentrated Oil

### (1) Preparing dosing samples

Using a 1.5% (by weight) aqueous solution of a fatty acid ester of glycerol (RYOTO® POLYGLYESTER; product of Mitsubishi-Kagaku Foods Corporation) as a solvent, an oil as a triglyceride of palmitoleic acid (C16:1) in concentrated form (product of KOYO fine chemical corporation) and olive oil (product of Sigma, with a purity of 99% and more) were added to the solvent and uniformly emulsified by sonication in an ice bath to thereby prepare dosing samples. The composition of major fatty acids in each of the oil as concentrated palmitoleic acid and the olive oil is shown in Table 1.

[Table 1]

**Table 1. Composition of Major Fatty Acids in each of Palmitoleic Acid Concentrated Oil and Olive Oil**

| Fatty acid (%) | Palmitoleic acid concentrated oil | Olive oil |
|---|---|---|
| C14:0 | 3.5 | 0.01 |
| C16:0 | 22.4 | 9.3 |
| C16:1 n-7 | 65.2 | 0.6 |
| C18:0 | 0.07 | 1.5 |
| C18:1 n-9 | 0.8 | 79.3 |
| C18:2 n-6 | 0.07 | 5.7 |

| | | |
|---|---|---|
| Values in the table are based on the average for samples subjected to three independent measurements. | | |

### (2) Experimental animal

Male Sprague-Dawley rats (hereinafter referred to as SD rats) were used in the test. Nine-week-old SD rats (Japan SLC, Inc.) were purchased and preliminarily reared for a week in individual cages. During the preliminary rearing period, the animals were allowed free access to the solid feed Labo MR Stock (Nosan Corporation) and distilled water through water bottles.

### (3) Main test with SD rats

After the preliminary rearing, the 10-week-old SD rats were divided into the following three groups (10 animals per group) considering their body weight: a control group administered with only the solvent (hereinafter referred to as "control group"); a group administered with the oil as a triglyceride of palmitoleic acid (C16:1) in concentrated form (hereinafter "C16:1 concentrated oil administered group"); and a group administered with olive oil (hereinafter "olive oil administered group"); these groups of rats were subjected to the main test. In the main test, the solvent or each oil under test was orally administered in a single dose through a gastric tube on the very day of the experiment. To the control group of SD rats, the solvent was orally administered at a dose of 10 mL/kg per animal. The test subject C16:1 concentrated oil was orally administered at such doses that the content of C16:1 in the C16:1 concentrated oil was 150 mg/kg or 500 mg/kg whereas olive oil was orally administered at such doses that the content of C18:1 in the olive oil was 150 mg/kg or 500 mg/kg. The amount of food intake (the amount of food supplied immediately after the dosing of sample minus the amount of food at a specified time after the dosing of sample) was measured an hour after oral administration of the test sample.

### (4) The effect on food intake of single-dose oral administration of the oil as triglyceride palmitoleate in concentrated form

An hour after administration of each test substance, the palmitoleic acid concentrated oil administered group (the amount of palmitoleic acid being equivalent to 500 mg/kg) ingested a significantly smaller amount of food than the control group. On the other hand, there were observed no significant decreases in the amount of food ingested by the olive oil administered group (Fig. 12).

### [Example 4] Study of the Effect of Short-Term Administration of Palmitoleic Acid (Free Fatty Acid) on Blood Cholecystokinin (CCK) Level and Small Intestine CCK mRNA Expression Level in SD Rats (Male)

### (1) Preparing dosing samples

Using a 1.5% (by weight) aqueous solution of a fatty acid ester of glycerol (RYOTO® POLYGLYESTER; product of Mitsubishi-Kagaku Foods Corporation) as a solvent, a free fatty acid form of palmitoleic acid (C16:1) and a free fatty acid form of oleic acid (C18:1), both being products of Sigma, were added to the solvent and uniformly emulsified by sonication in an ice bath to thereby prepare dosing samples.

### (2) Experimental animal

Male Sprague-Dawley rats (hereinafter referred to as SD rats) were used in the test. Nine-week-old SD rats (Japan SLC, Inc.) were purchased and preliminarily reared for a week in individual cages. During the preliminary rearing period, the animals were allowed free access to the solid feed Labo MR Stock (Nosan Corporation) and distilled water through water bottles.

### (3) Main test with SD rats

After the preliminary rearing, the 10-week-old SD rats were divided into the following three groups (10 animals per group) considering their body weight: a control group administered with only the solvent (hereinafter referred to as "control group"); a group administered with palmitoleic acid (C16:1) (hereinafter "C16:1 administered group"); and a group administered with oleic acid (C18:1) (hereinafter "C18:1 administered group"); these groups of rats were subjected to the main test. In the main test, the solvent or each fatty acid under test was orally administered in a single dose through a gastric tube on the very day of the experiment. To the control group of SD rats, the solvent was orally administered at a dose of 10 mL/kg per animal. The test subjects, palmitoleic acid and oleic acid, each weighing 150 mg and 500 mg, were added to 10 mL of the solvent, emulsified and orally administered to the SD rats at a dose of 10 mL/kg per animal; an hour later, the animals were exsanguinated from the ventral aorta under 4% pentobarbital anesthesia in the presence of heparin and bled to death. Using a centrifuge (CF8DL of Hitachi Koki Co., Ltd.), the collected blood was centrifugally separated (4 °C, 3000 rpm (ca. 1972 g), 15 min); the obtained blood plasma was stored frozen until measurement of blood cholecystokinin (CCK) level. The blood level of CCK was measured with an enzyme immunoassay (ELISA) kit (Cholecystokinin (CCK) EIA Kit, Phoenix Pharmaceuticals, Inc.) In addition, the expression level of CCK messenger RNA (mRNA) was measured and evaluated by performing real-time PCR reaction on cDNA synthesized for the total RNA extracted from the small intestine. As an endogenous control gene, 18s ribosomal RNA gene was used. With the level of gene expression in the control group taken as unity, the relative expression levels of respective genes in the C16:1 administered group or 18:1 administered group were calculated. The primers for the CCK gene were as follows:
F 5'-CATCCAGCAGGTCCGCAAA-3', R 5'-TCCATCCAGCCCATGTAGTCC-3'.

### (4) Results

An hour after administration of each test substance, the blood CCK level and the small intestine CCK mRNA expression level in the C16:1 administered group (500 mg/kg) increased significantly in comparison with the corresponding levels in the control group but no significant elevation of the blood CCK level was observed in the oleic acid administered group (Figs. 13 and 14).

## Claims

1. An anorectic agent comprising a component selected from among palmitoleic acid, salts thereof, and esters thereof as an active ingredient.

2. The anorectic agent according to claim 1, wherein the palmitoleic acid esters are selected from C₁₋₆ alkyl esters of palmitoleic acid and glycerides comprising palmitoleic acid as a constituent fatty acid.

3. The anorectic agent according to claim 1, which comprises an ethyl ester of palmitoleic acid as the active ingredient.

4. The anorectic agent according to claim 1, which comprises as the active ingredient a triglyceride comprising palmitoleic acid as a constituent fatty acid.

5. The anorectic agent according to claim 4, wherein palmitoleic acid accounts for 30% or more of the fatty acid composition of the triglyceride.

6. A pharmaceutical composition for use in treatment or prevention of eating disorder or obesity, which comprises a component selected from among palmitoleic acid, salts thereof, and esters thereof as an active ingredient.


SEQUENCE LISTING

<110>  Nippon Suisan Kaisha, Ltd.

<120>  Anorectic Agent

<130>  FA0066-12088PCT

<150>  Japanese Patent Application No. 2012-9387
<151>  2012-01-19

<160>  2

<170>  PatentIn version 3.5

<210>  1
<211>  19
<212>  DNA
<213>  rat

<400>  1
catccagcag gtccgcaaa                                                    19


<210>  2
<211>  21
<212>  DNA
<213>  rat

<400>  2
tccatccagc ccatgtagtc c                                                 21